(19) Europäisches Patentamt
European Patent Office
Office européen des brevets

(11) Veröffentlichungsnummer: **0 005 231**
**B1**

(12) **EUROPÄISCHE PATENTSCHRIFT**

(45) Veröffentlichungstag der Patentschrift: **15.12.82**

(21) Anmeldenummer: **79101232.1**

(22) Anmeldetag: **24.04.79**

(51) Int. Cl.$^3$: **C 07 D 217/22,**
C 07 D 217/26,
C 07 D 403/04,
C 07 D 403/06,
C 07 D 409/04,
A 61 K 31/47

(54) **Isochinolinderivate, Verfahren zu ihrer Herstellung und diese enthaltende pharmazeutische Zubereitungen.**

(30) Priorität: **27.04.78 DE 2818423**

(43) Veröffentlichungstag der Anmeldung:
**14.11.79 Patentblatt 79/23**

(45) Bekanntmachung des Hinweises auf die Patenterteilung:
**15.12.82 Patentblatt 82/50**

(84) Benannte Vertragsstaaten:
**CH DE FR GB**

(56) Entgegenhaltungen:
**AU - B - 4 993 472**
**DE - A - 2 030 675**
**DE - A - 2 503 961**

(73) Patentinhaber: **HOECHST AKTIENGESELLSCHAFT**
**Postfach 80 03 20**
**D-6230 Frankfurt/Main 80 (DE)**

(72) Erfinder: **Bartmann, Wilhelm, Dr.**
**Am Dachsbau 5**
**D-6232 Bad Soden am Taunus (DE)**
Erfinder: **Konz, Elmar, Dr.**
**Buchenweg 22**
**D-6232 Bad Soden am Taunus (DE)**

**EP 0 005 231 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Isochinolinderivate, Verfahren zu ihrer Herstellung und diese enthaltende pharmazeutische Zubereitungen

Die Erfindung betrifft neue substituierte Isochinolinderivate mit wertvollen pharmakologischen, insbesondere psychotropen, antiarrhytmischen und antiphlogistischen Eigenschaften.

Gegenstand der Erfindung sind daher Isochinolinderivate der allgemeinen Formel I

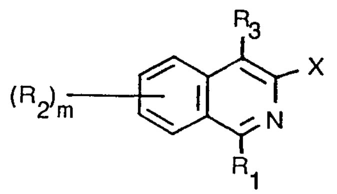

(I)

worin bedeuten

m gleich eins oder zwei

X Brom oder Chlor

$R_1$ einen Phenylrest, der gegebenenfalls mit Halogen-, Hydroxy-, Nitro-, Amino-, oder einer durch ein oder zwei aliphatische, cycloaliphatische oder aromatische Kohlenwasserstoffreste substituierten Aminogruppe mit zwei bis achtzehn Kohlenstoffatomen, wobei das Stickstoffatom auch in einen heterocyclischen Ring eingeschlossen sein kann, einer Acylamino-, Alkyl- oder Alkoxygruppe mit jeweils einem bis sechs Kohlenstoffatomen, einer Benzyloxy- oder einer Trifluormethylgruppe mono- oder disubstituiert ist, einen Pyridyl- oder Thienylrest.

$R_2$ Wasserstoff, Halogen-, Hydroxy-, Alkyl- oder Alkoxygruppe mit einem bis sechs Kohlenstoffatomen, Nitro-, Amino-, Benzyloxy-, Methylendioxy- oder Äthylendioxygruppe.

$R_3$ eine Carboxyl-, Cyano-, Hydroxymethyl-, eine Alkoxymethylgruppe mit 1 bis 6 Kohlenstoffatomen, eine Aminomethylgruppe der Formel

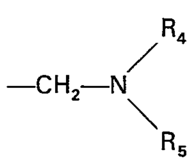

worin $R_4$ und $R_5$ gleich oder verschieden sind und Wasserstoff oder einen geradkettigen oder verzweigten, gesättigten oder ungesättigten Alkylrest mit 1 bis 8 Kohlenstoffatomen bedeuten, wobei die Alkylreste auch substituiert sein können mit Hydroxy-, $C_1$—$C_4$-Alkoxy oder einer Aminogruppe der Formel

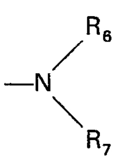

,

wobei $R_6$ und $R_7$ gleich oder verschieden sind und Wasserstoff oder einen geradkettigen oder verzweigten Alkylrest mit 1 bis 6 Kohlenstoffatomen, oder zusammen mit dem Stickstoffatom einen heterocyclischen Ring mit bis zu 7 Kohlenstoffatomen darstellen.

Die Alkylreste $R_4$ und $R_5$ können auch gemeinsam mit dem Stickstoffatom einen 5- bis 8-gliedrigen Ring bilden, wobei der heterocyclische Ring an einem Kohlenstoffatom substituiert sein kann mit einer $C_1$—$C_6$-Alkyl-, $C_1$—$C_4$-Alkoxy-, Hydroxy-, Carboxy- oder $C_1$—$C_4$-Alkoxycarbonylgruppe, und worin eines der Kohlenstoffatome durch ein Sauerstoff-, Schwefel- oder Stickstoffatom ersetzt sein kann, wobei das letzte substituiert sein kann durch Wasserstoff, die Thienyl-, Furyl-, Pyridyl- oder Formylgruppe, eine $C_3$—$C_8$ Alkenyloxycarbonyl- oder $C_3$—$C_8$-Alkinyloxycarbonylgruppe, eine gegebenenfalls durch Hydroxy- oder $C_1$—$C_4$-Alkoxygruppen substituierte $C_1$—$C_6$-Alkoxycarbonylgruppe, einen Phenylrest, der einfach oder mehrfach substituiert sein kann mit $C_1$—$C_4$-Alkyl-, $C_1$—$C_4$-Alkoxy, Methylendioxy-, Hydroxy-, Nitro- oder Aminogruppe oder Halogen, und wobei das Wasserstoffatom am Stickstoff weiterhin ersetzt sein kann durch den Rest —$COR_8$, worin $R_8$ einen Thienyl-, Furyl-, Pyridylrest oder einen gegebenenfalls wie oben angegebenen substituierten Phenylrest, oder eine $C_1$—$C_6$-Alkylgruppe bedeutet, die ihrerseits substituiert sein kann mit Hydroxy, $C_1$—$C_4$-Alkoxy-, $C_1$—$C_6$-Dialkylamino-, Äthylendioxy-, Trimethylendioxygruppe oder einen gegebenenfalls wie oben angegebenen substituierten Phenylrest. Weiterhin kann $R_3$ eine Carbamidgruppe der Formel

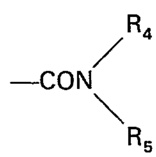

sein, worin $R_4$ und $R_5$ die obengenannte Bedeutung haben, sowie deren physiologisch verträglichen Salze, Verfahren zur Herstellung der Verbindungen und pharmazeutische Zubereitungen.

Insbesondere beeinhaltet die Erfindung Verbindungen, worin m gleich eins oder zwei bedeutet, X gleich Chlor ist, $R_1$ einen Phenylrest, der gegebenenfalls mit Halogen-, Hydroxy-, Nitro-, Amino-, oder eine durch ein oder zwei aliphatische, cycloaliphatische oder aromatische Kohlenwasserstoffatome substituierten Aminogruppe mit zwei bis zwölf Kohlenstoffatome, wobei das Stickstoffatom auch in einem heterocyclischen Ring eingeschlossen sein kann, eine Acylamino, Alkyl- oder Alkoxygruppe mit jeweils einem bis vier Kohlenstoffatomen, einer Benzyloxy- oder einer Trifluormethylgruppe, mono oder disubstituiert ist, oder

einen Pyridylrest, $R_2$ Wasserstoff, Halogen, Hydroxy, Nitro, Amino oder Alkyl- oder Alkoxygruppe mit 1 bis 4 Kohlenstoffatomen und $R_3$ eine Carboxyl-, Cyano-, Hydroxymethyl, eine Alkoxymethylgruppe mit 1 bis 4 Kohlenstoffatomen, eine Aminomethylgruppe der Formel

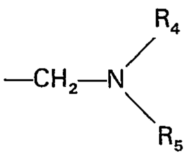

oder eine Carbamidgruppe der Formel

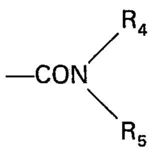
,

worin $R_4$ und $R_5$ gleich oder verschieden sind und Wasserstoff, einen geradkettigen oder verzweigten gesättigten oder ungesättigten Alkylrest mit 1 bis 4 Kohlenstoffatomen darstellen, wobei die Alkylreste auch gemeinsam mit dem Stickstoffatom einen 5 bis 7-gliedrigen Ring bilden können, worin eines der Kohlenstoffatome durch ein Sauerstoff-, Schwefel- oder Stickstoffatom ersetzt sein kann, wobei das letztere substituiert sein kann durch Wasserstoff, den Thienyl-, Furyl-, Pyridyl- oder Formylgruppe, eine gegebenenfalls durch Hydroxy- oder $C_1$—$C_4$-Alkoxygruppen substituierte $C_1$—$C_4$-Alkoxycarbonylgruppe, den Phenylrest der einfach oder mehrfach substituiert sein kann mit $C_1$—$C_4$-Alkyl-, $C_1$—$C_4$-Alkoxy-, Methylendioxy-, Hydroxy-, Nitro- oder Aminogruppe oder Halogen und wobei das Wasserstoffatom am Stickstoff weiterhin ersetzt sein kann durch den Rest —$COR_8$, worin $R_8$ einen Thienyl-, Furyl-, Pyridylrest oder einen gegebenenfalls wie oben angegebenen substituierten Phenylrest bedeutet, oder durch eine $C_1$—$C_4$-Alkylgruppe die ihrerseits substituiert sein kann durch Hydroxy, $C_1$—$C_4$-Alkoxy-, $C_1$—$C_4$-Dialkylamino oder den gegebenenfalls wie oben angegebenen substituierten Phenylrest, worin weiterhin, wenn $R_4$ Wasserstoff oder $C_1$—$C_4$-Alkyl ist, $R_5$ einen Aminoalkylrest der Formel

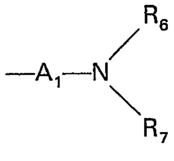

bedeutet, worin $A_1$ eine geradkettige oder verzweigte $C_2$—$C_6$-Alkylengruppe darstellt, die durch Hydroxy- oder $C_1$—$C_4$-Alkoxygruppe substituiert sein kann und worin $R_6$ und $R_7$ die obengenannte Bedeutung haben.

Von ganz besonderem Interesse sind Verbindungen, worin m gleich eins oder zwei bedeutet, X gleich Chlor ist, $R_1$ einen Phenylrest, der gegebenenfalls mit Halogen, Hydroxy-, Nitro, Amino, $C_1$—$C_4$-Alkyl-, Methoxy- oder Trifluormethylgruppen mono- oder disubstituiert ist, $R_2$ Wasserstoff, Halogen, Hydroxy-, Nitro-, Amino oder $C_1$—$C_4$-Alkyl- oder eine Alkoxygruppe mit 1 bis 4 Kohlenstoffatomen und $R_3$ eine Carboxyl-, Cyano-, Hydroxymethyl oder Aminomethylgruppe der Formel

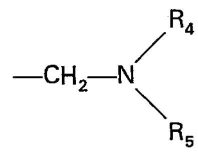

oder eine Carbamidgruppe der Formel

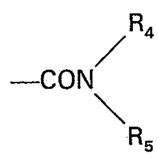

bedeutet, in denen die Alkylreste $R_4$ und $R_5$ gemeinsam mit dem Stickstoffatom einen 5- bis 7-gliedrigen Ring bilden und worin eines der Kohlenstoffatome durch ein N- oder O-Atom ersetzt sein kann, insbesondere den Pyrrolidino-, Piperidino-, Hexamethylenimino-, Morpholino-, 4-Hydroxypiperidino-, 4-Carbäthoxypiperidino- und den 1-Piperazinylrest

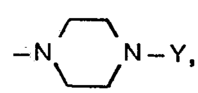
,

worin Y Wasserstoff, $C_1$—$C_4$-Alkyl, $\beta$-Hydroxyäthyl, 3,4-Methylendioxybenzyl, Phenyl, durch Methoxy, Chlor, Nitro oder Amino substituiertes Phenyl, 3,4,5-Trimethoxybenzoyl, 3,4-Methylendioxybenzoyl, 2-Furoyl, 2-Thienoyl, $C_1$—$C_3$-Alkoxycarbonyl bedeutet, wobei der Alkylrest im letzteren durch OH, Methoxy oder Äthoxy substituiert sein kann, oder wenn $R_4$ Wasserstoff oder $C_1$—$C_4$-Alkyl ist, $R_5$ einen Aminoalkylrest der Formel

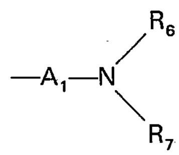

bedeutet, worin $A_1$, $R_6$ und $R_7$ die oben genannte Bedeutung haben.

Gegenstand der Erfindung sind weiterhin Verfahren zur Herstellung sowie pharmazeutische Zubereitungen dieser Verbindungen.

Das Verfahren zur Herstellung der Verbindungen der allgemeinen Formel I ist dadurch gekennzeichnet, daß man

a) Verbindungen der allgemeinen Formel II

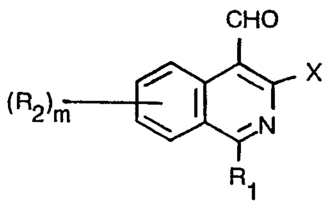

(II)

worin X, $R_1$, $R_2$ und m die zur allgemeinen Formel I genannte Bedeutung haben, zu Verbindungen der allgemeinen Formel I oxidiert, worin $R_3$ eine Carboxylgruppe ist. Diese können gegebenenfalls mit einem Amin

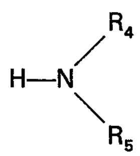

,

worin $R_4$ und $R_5$ die zur allgemeinen Formel I genannte Bedeutung haben, zu Verbindungen der allgemeinen Formel I, in denen $R_3$ eine Carbamidgruppe der Formel

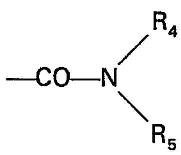

darstellt, umgesetzt werden.

b) Verbindungen der allgemeinen Formel III

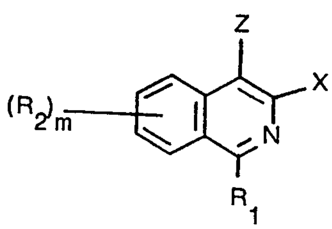

(III)

worin Z eine Formyl-, Nitril-, Carboxy-, Halogencarboxyl- oder eine Alkylcarboxylgruppe mit 1 bis 7 Kohlenstoffatomen bedeutet und X, $R_1$, $R_2$ und m die zur allgemeinen Formel I genannte Bedeutung haben, zu Verbindungen der allgemeinen Formel I reduziert, worin $R_3$ eine Hydroxymethylgruppe darstellt.

c) Verbindungen der allgemeinen Formel IV

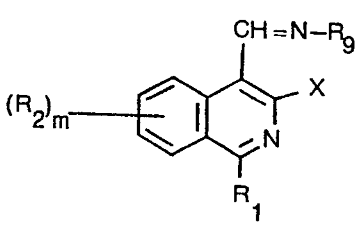

(IV)

worin X, $R_1$, $R_2$ und m die zur allgemeinen Formel I genannte Bedeutung haben, zu Verbindungen der allgemeinen Formel I reduziert, worin $R_3$ die Methylenaminogruppe der Formel $-CH_2-NH-R_9$ und $R_9$ der Rest

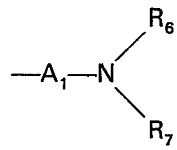

sein soll, wobei $A_1$, $R_6$ und $R_7$ die zur allgemeinen Formel I genannte Bedeutung haben, darstellt,

d) Verbindungen der allgemeinen Formel V

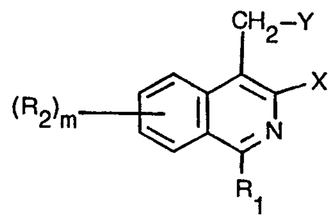

(V)

worin Y gleich Chlor, Brom oder Hydroxyl und X, $R_1$, $R_2$ und m die zur allgemeinen Formel I genannte Bedeutung haben, mit einem Amin der Formel

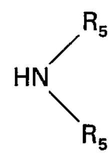

,

worin $R_4$ und $R_5$ die zur allgemeinen Formel I genannte Bedeutung haben, worin $R_3$ gleich

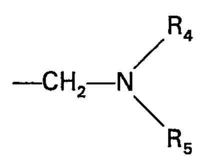

ist, umsetzt,

e) Verbindungen der allgemeinen Formel VI

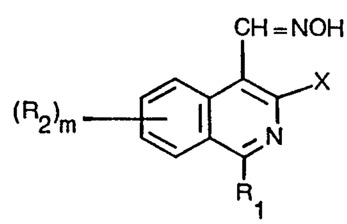

(VI)

worin X, $R_1$, $R_2$ und m die zur allgemeinen Formel I genannte Bedeutung haben, mit wasserabspaltenden Mitteln in Verbindungen der allgemeinen Formel I überführt, worin $R_3$ eine Nitrilgruppe darstellt.

f) Verbindungen der allgemeinen Formel VII

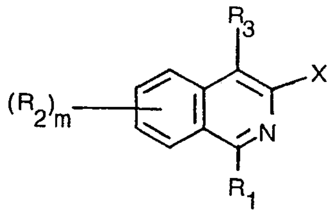

(VII)

worin X, $R_1$, $R_2$, $R_3$ und m die zur allgemeinen Formel I genannte Bedeutung haben, mit der Maßgabe, daß in je einem oder beiden Resten $R_1$ und $R_3$ eine sekundäre Aminogruppe enthalten ist, mit einem Alkylierungsmittel der Formel $Y—R_{10}$, worin Y Jod, Chlor oder Brom, und $R_{10}$ einen geradkettigen oder verzweigten $C_1—C_6$-Alkylrest, der durch Hydroxy-$C_1—C_4$-Alkoxy, $C_1—C_4$-Dialkylamino, Äthylendioxy, Trimethylendioxy oder auch gegebenenfalls substituiertes Phenyl substituiert sein kann, oder einen $C_3—C_8$-Alkenyl- oder $C_3—C_8$-Alkinylrest bedeutet, oder mit einem Chlorameisensäureester der Formel

$$Cl—CO_2—(C_1—C_4)\text{-alkyl},$$

wobei die Alkylreste Hydroxy- oder $C_1—C_4$-Alkoxygruppen tragen oder mit einer Verbindung der Formel $Cl—COR_8$, worin $R_8$ die zur allgemeinen Formel I genannte Bedeutung hat, umsetzt,

g) Verbindungen der allgemeinen Formel I

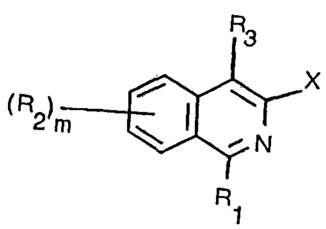

(I)

worin X, $R_1$, $R_2$, $R_3$ und m die zur allgemeinen Formel I genannte Bedeutung haben und sofern $R_1$ den Phenylring darstellt diesen Phenylring nachträglich entsprechend substituiert.

Bei dem *Verfahren a)* werden die Verbindungen II nach bekannten Methoden, z.B. mit Mangandioxyd, Kaliumpermanganat, oxidiert. Derartige Oxydationsreaktionen sind bekannt. [vgl. "Compendium of Organic Synthetic Methods", Verlag John Winley u. Sons, Inc. (1971), Seiten 32—26]. Die Carbonsäuren werden nach den üblichen Methoden der Amidbildung z.B. über die Säurechloride, in die Amide übergeführt. Die Ausgangsverbindungen II für das Verfahren a) können gemäß der deutschen Patentanmeldung P 28 11 361.3 hergestellt werden z.B. durch Umsetzung von Verbindungen der allgemeinen Formel VIII

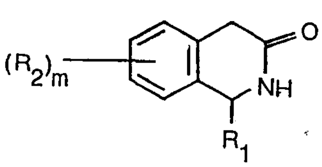

(VIII)

worin $R_1$, $R_2$ und m die zur allgemeinen Formel I genannte Bedeutung haben mit einem Vilsmeier-Addukt aus einem Säureamid mit einem Säurechlorid oder -bromid zu Verbindung der allgemeinen Formel IX

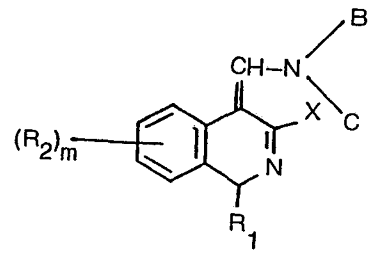

(IX)

worin X gleich Chlor oder Brom ist und B und C Alkyl oder Cycloalkyl mit einem bis sechs Kohlenstoffatomen oder Phenyl bedeuten und anschließender Oxidation zu Verbindungen der allgemeinen Formel II.

Bei dem *Verfahren b)* werden die Verbindungen I und II nach bekannten Methoden reduziert. Als Reduktionsmittel kommen komplexe Metallhydride, z.B. Natriumborhydrid, Lithiumaluminiumhydrid und als Lösungsmittel Methanol, Äthanol, Tetrahydrofuran, Dimethoxyäthan in Betracht.

Bei dem *Verfahren c)* werden die Verbindungen IV nach bekannten Methoden reduziert. Als Reduktionsmittel kommen komplexe Metallhydride wie Natriumborhydrid, Lithiumaluminiumhydrid und als Lösungsmittel Methanol, Äthanol, Tetrahydrofuran, Dimethoxyäthan in Frage. Die Verbindungen IV können aus den Verbindungen II durch Umsetzung mit einem Amin unter Säurekatalyse erhalten werden. Dabei kann das entstehende Reaktionswasser vorzugsweise mittels eines Wasserabscheiders abgetrennt werden.

Bei dem *Verfahren d)* werden die Verbindungen V mit einem Amin in Gegenwart eines Halogenwasserstoff bindenden Mittels umsetzt. Als Halogenwasserstoff bindendes Mittel kommen ein Überschuß des Amins selbst, tertiäre Amine wie Triäthylamin, Pyridin oder auch Kaliumcarbonat oder ähnliche in Frage und als Lösungsmittel, sofern sie zur Umsetzung verwendet werden, können indifferente, wasserfreie organische Lösungsmittel wie Dioxan, Dimethoxyäthan, Diäthylenglykoldimethyläther, Diäthylenglykoldibutyläther, Benzol, Toluol, Xylol, Chlorbenzol, Dimethylformamid, Dimethylsulfoxid oder Hexamethylphosphorsäuretriamid eingesetzt werden. Die Reaktion wird im allgemeinen bei einer Temperatur zwischen 20 und 180°C vorzugsweise zwischen 20 und 130°C ausgeführt. Die für das Verfahren g) benötigten Verbindungen V, bei denen Y gleich Chlor oder Brom ist, können durch Umsetzung der Verbindungen V, bei denen Y gleich Hydroxyl ist, mit Halogenierungsmitteln, wie Phosphortrichlorid, Phosphortribromid, nach bekannten Methoden dargestellt werden.

Bei dem *Verfahren e)* kommen als wasserabspaltende Mittel z.B. Phosphorpentoxyd, Phosphoroxychlorid, Essigsäureanhydrid in Frage. Die Reaktion wird im allgemeinen bei einer Temperatur zwischen 50 und 150°C ausgeführt und als Lösungsmittel können Pyridin, Benzol, Toluol oder N,N-Dimethylformamid verwendet werden.

Nach dem *Verfahren f)* werden sekundäre Aminogruppen nach an sich bekannten Methoden mit dem Alkylierungsmittel Y—$R_{10}$ alkyliert.

Nach dem *Verfahren g)* können in den aromatischen Rest $R_4$ durch elektrophile Substitution Substituenten eingeführt werden. Dazu gehören vor allem die Halogenierung, Sulfonierung oder Nitrierung wobei der Nitrierung besonderes Interesse zukommt. Man geht dabei so vor, daß man Verbindungen der allgemeinen Formel I den üblichen Nitrierbedingungen unterwirft (Schwefelsäure, Salpetersäure, Eiskühlung).

Die erfindungsgemäßen Verbindungen haben wertvolle therapeutische Eigenschaften. So zeigen sie neben anderen pharmakologischen Eigenschaften eine Wirkung auf das Zentralnervensystem. Sie können die durch den elektrischen Strom oder Pentamethylentetrazol ausgelösten Krämpfe verhindern und verlängern die Thiopental- bzw. Hexabarbitalnarkose. Auf Grund all dieser Eigenschaften können die erfindungsgemäßen Verbindungen als Wirkstoffe von sedierend, tranquilisierend und krampfhemmend wirkenden Arzneimitteln angewandt werden.

Außerdem zeigen die erfindungsgemäßen Verbindungen eine Wirkung auf dem Herz-Kreislauf, die sich insbesondere in einer antiarrhythmischen Aktivität äußert. Damit eignen sich diese Verbindungen für die Behandlung von Herz-Rhythmus-Störungen. Die antiarrhythmische Aktivität wurde am strophantinvergifteten Hund nachgewiesen.

Daneben besitzen die erfindungsgemäßen Verbindungen antiphlogistische Wirkung, die im Adjuvans-Arthritis Modell nachgewiesen wurde.

Die neuen Verbindungen können entweder allein oder mit physiologisch verträglichen Hilfs- oder Trägerstoffen vermischt angewandt werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige, alkoholische oder ölige Lösungen. Als inerte Träger können z.B. Magnesiumcarbonat, Milchzucker oder Maisstärke verwendet werden. Dabei kann die Zubereitung sowohl als Trocken- oder Feuchtgranulat erfolgen. Als ölige Trägerstoffe oder Lösungsmittel kommen beispielsweise besonders pflanzliche und tierische Öle in Betracht wie z.B. Sonnenblumenöl oder Lebertran.

Eine besondere Anwendungsform liegt in der intravenösen Applikation. Zu diesem Zweck werden die aktiven Verbindungen oder deren physiologisch verträglichen Salze mit den dafür üblichen Substanzen in Lösung gebracht. Solche physiologisch verträglichen Salze werden z.B. mit folgenden Säuren gebildet: Chlor-, Brom- oder Jodwasserstoffsäure, Phosphorsäure, Schwefelsäure, Methylschwefelsäure, Amidosulfonsäure, Salpetersäure, Ameisensäure, Essigsäure, Propionsäure, Bernsteinsäure, Weinsäure, Milchsäure, Malonsäure, Fumarsäure, Oxalsäure, Citronensäure, Apfelsäure, Schleimsäure, Benzoesäure, Salicylsäure, Acetursäure, Embonsäure, Naphthalin-1,5-Disulfonsäure, Ascorbinsäure, Phenylessigsäure, p-Aminosalicylsäure, Hydroxyäthansulfonsäure, Benzolsulfonsäure oder synthetische Harze, die saure Gruppen enthalten, z.B. solche mit Ionenaustauscherwirkung. Als Lösungsmittel der entsprechenden physiologisch verträglichen Salze der aktiven Verbindungen für eine intravenöse Applikation kommen z.B. in Frage: Wasser, physiologische Kochsalzlösungen oder Alkohole, wie z.B. Äthanol, Propandiol oder Glycerin, daneben auch Zuckerlösungen wie z.B. Glukose- oder Mannitlösungen, oder auch eine Mischung aus den verschiedenen genannten Lösungsmitteln.

Die erfindungsgemäßen Verbindungen der allgemeinen Formel I sind zu vielfältigen weiteren Reaktionen fähig und daher wertvolle Zwischenprodukte für Arzneimittel.

Beispiel 1

3-Chlor-1-phenyl-isochinolin-4-carbonsäure

53,5 g 3-Chlor-1-phenyl-isochinolin-4-aldehyd wird in 1,5 l Aceton und 500 ml Phosphatpuffer von pH 7 suspendiert. Bei 40°C trägt man portionsweise im Laufe von 2 Stunden 40 g Kaliumpermanganat ein und rührt bei dieser Temperatur 2 Stunden nach. Das überschüssige Kaliumpermanganat wird mit 10 g Natriumhydrogensulfit zerstört und die Lösung auf 500 ml einrotiert und filtriert. Das Filtrat wird mit konzentrierter Salzsäure auf pH 4 gebracht und mit Essigester gründlich extrahiert. Nach dem Entfernen des Lösungsmittels im Vakuum verbleiben 41,1 g 3-Chlor-1-phenyl-isochinolin-4-carbonsäure mit Schmp. 208°C.

Die folgenden Carbonsäuren der Tabelle 1 werden aus den entsprechenden Aldehyden gemäß Beispiel 1 erhalten.

TABELLE 1

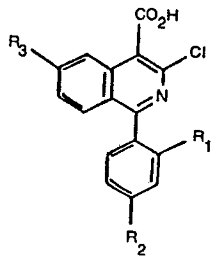

| Beispiel | $R_1$ | $R_2$ | $R_3$ | Schmp. °C |
|---|---|---|---|---|
| 2 | H | H | Cl | 268—271 |
| 3 | H | Cl | H | 230—237 |
| 4 | $CH_3$ | H | H | 105 |
| 5 | F | H | H | 180—184 |

Beispiel 6

3-Chlor-1-phenyl-isochinolin-4-carbonsäure-N-methylpiperazid

Zu 11,3 g N-Methylpiperazin in 100 ml Chloroform werden bei Raumtemperatur 11,3 g 3-Chlor-1-phenyl-isochinolin-4-carbonsäurechlorid getropft. Nach 6 Stunden bei Raumtemperatur wird das Lösungsmittel entfernt und der Rückstand mit gesättigter Natriumhydrogencarbonatlösung verrührt. Es werden 13,3 g des N-Methylpiperazid mit Schmp. 164—167°C isoliert.

Hydrochlorid 256°C.

Das 3-Chlor-1-phenyl-isochinolin-4-carbonsäurechlorid wird aus der entsprechenden Carbonsäure durch 4-stündiges Kochen mit überschüssigem Thionylchlorid dargestellt. Nach dem Entfernen des überschüssigen Thionylchlorid verbleibt das rohe Säurechlorid und wird sofort weiterverarbeitet.

Analog zu Beispiel 6 werden aus den entsprechenden Säurechloriden und Aminen die Verbindungen der Tabelle 2 dargestellt.

TABELLE 2

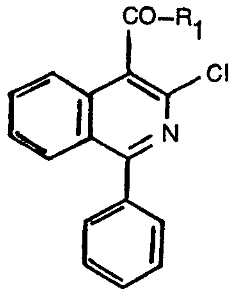

| Beispiel | $R_1$ | Schmp. °C | Salz (Schmp. °C) |
|---|---|---|---|
| 7 | $-N(CH_3)_2$ | 108 | |
| 8 | 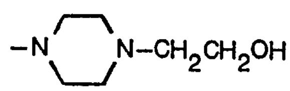 | 146–148 | HCl (256–258) |
| 9 | 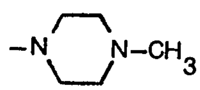 | 164–167 | HCl (255–256) |
| 10 | 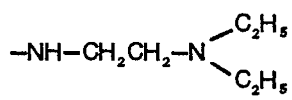 | Öl | Oxalat (167–169) |
| 11 | 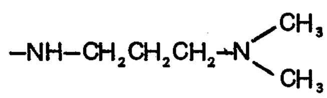 | 105–108 | HCl (207–208) |
| 12 | 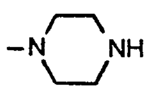 | 158–165 | HCl (240–244) |

Beispiel 13

3-Chlor-4-cyano-1-phenyl-isochinolin

53,6 g 3-Chlor-1-phenyl-isochinolin-4-aldehyd in 150 ml Pyridin wird bei 0°C mit 55,6 g Hydroxylaminhydrochlorid versetzt und nach 2 Stunden bei Raumtemperatur das Lösungsmittel im Vakuum entfernt. Der Rückstand wird zwischen Wasser und Toluol verteilt. Die Toluolphase getrocknet und einrotiert. Man erhält 43,2 g 3-Chlor-1-phenyl-isochinolin-4-aldoxim mit Schmp. 151—154°C.

Zur Überführung in die 4-Cyano-Verbindungen werden 67,3 g Aldoxim in 700 ml Pyridin bei 0°C mit 91,4 g Phosphoroxychlorid versetzt und 12 Stunden bei Raumtemperatur stehen gelassen. Das Reaktionsgemisch wird mit Wasser hydrolysiert und der kristalline Niederschlag abfiltriert. Nach dem Umlösen aus Äthanol erhält man 47 g 3-Chlor-4-cyano-1-phenyl-isochinolin mit Schmp. 191—193°C.

Beispiel 14

4-Aminomethyl-3-chlor-1-phenyl-isochinolin

12,5 g 3-Chlor-1-phenyl-isochinolin-4-aldoxim werden in 500 ml methanolischem Ammoniak mit 30 g vorhydriertem Raney-Nickel bei Raumtemperatur und Atmosphärendruck hydriert. Nach 30 Minuten wird vom Katalysator abfiltriert die Lösung eingeengt und der Rückstand an 200 g Kieselgel mit einer Chloroform-Methanol-Mischung (8:2) chromatographiert. Man isoliert 2,5 g 4-Amino-

methyl-3-chlor-1-phenyl-isochinolin mit Schmp. 101—103°C.
Hydrochlorid 295°C.

Beispiel 15

3-Chlor-1-(4-chlorphenyl)-6,7-dimethoxy-4-(3-dimethylaminopropyl)-aminomethyl-isochinolin

3,62 g 3 - Chlor - 1 - (4 - chlorphenyl) - 6,7-dimethoxy-isochinolin-4-aldehyd wird mit 3,06 g 3-Dimethylaminopropylamin in 100 ml DMF und 50 ml $CH_3OH$ 4 Stunden auf 80°C erhitzt. Das Lösungsmittel wird im Vakuum entfernt, der Rückstand in 150 ml wasserfreiem Methanol gelöst und bei 10°C portionsweise mit 1,53 g Natriumborhydrid versetzt. Die Reaktionsmischung wird 3 Stunden nachgerührt, im Vakuum das Lösungsmittel entfernt und der Rückstand mit Wasser verrührt. Man erhält 4,2 g 3 - Chlor - 1 - (4 - chlorphenyl) - 6,7-dimethoxy - 4 - (3 - dimethylaminopropyl)-aminomethyl-isochinolin mit Schmp. 126—127°C.
Dihydrochlorid: 250—252°C.

Beispiel 16

3-Chlor-4-hydroxymethyl-1-phenyl-isochinolin

8,1 g 3 - Chlor - 1 - phenyl - isochinolin - 4-aldehyd in 150 ml Methanol werden bei 0°C mit 1,8 g Natriumborhydrid portionsweise versetzt. Die Lösung wird bei Raumtemperatur 4 Stunden nachgerührt und das Lösungsmittel im Vakuum entfernt. Der Rückstand wird mit Wasser verrührt und 7,2 g 3-Chlor-4-hydroxymethyl-1-phenyl-isochinolin mit Schmp. 175—182°C abfiltriert.

Beispiel 17

3 - Chlor - 4 - dimethylaminomethyl - 1 - phenyl - isochinolin

8,2 g 3-Chlor-4-hydroxymethyl-1-phenyl-isochinolin werden in 300 ml Toluol suspendiert und unter Eiskühlung mit 16,2 g Phosphortribromid versetzt. Nach 10 Stunden Rühren bei Raumtemperatur wird Dimethylamin eingeleitet bis zur alkalischen Reaktion. Das Reaktionsgemisch wird eingeengt und der Rückstand mit Wasser versetzt. Die Extraktion mit Essigester ergibt ein gelbes Öl, das langsam kristallisiert, Schmp. 75—77°C.
Hydrochlorid 244—246°C.

Beispiel 18

3-Chlor-1-phenyl-isochinolin-4-carbonsäure-N-methylpiperazid

7,0 g 3 - Chlor - 1 - phenyl - isochinolin - 4-carbonsäurepiperazid, 3,5 g Methyljodid und 3,6 g Natriumcarbonat werden in 100 ml Toluol 3 Stunden unter Rückfluß gekocht. Der anorganische Niederschlag wird abfiltriert und das Lösungsmittel im Vakuum entfernt. Es werden 6,2 g N - Methyl - piperazid mit Schmp. 164—167°C isoliert.

**Patentansprüche**

1. Isochinolinderivate der allgemeinen Formel I

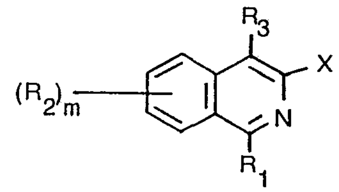

(I)

worin bedeuten:

m gleich eins oder zwei

X Brom oder Chlor

$R_1$ einen Phenylrest, der gegebenenfalls mit Halogen-, Hydroxy-, Nitro-, Amino-, oder einer durch ein oder zwei aliphatische, cycloaliphatische oder aromatische Kohlenwasserstoffreste substituierten Aminogruppe mit zwei bis achtzehn Kohlenstoffatomen, wobei das Stickstoffatom auch in einen heterocyclischen Ring eingeschlossen sein kann, einer Acylamino-, Alkyl- oder Alkoxygruppe mit jeweils einem bis sechs Kohlenstoffatomen, einer Benzyloxy- oder einer Trifluormethylgruppe mono- oder disubstituiert ist, einen Pyridyl- oder Thienylrest,

$R_2$ Wasserstoff, Halogen-, Hydroxy-, Alkyl- oder Alkoxygruppe mit einem bis sechs Kohlenstoffatomen, Nitro-, Amino-, Benzyloxy-, Methylendioxy- oder Äthylendioxygruppe,

$R_3$ eine Carboxyl-, Cyano-, Hydroxymethyl-, Alkoxymethylgruppe mit 1 bis 6 Kohlenstoffatomen, eine Aminomethylgruppe der Formel

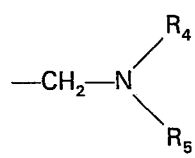

,

worin $R_4$ und $R_5$ gleich oder verschieden sind und Wasserstoff oder einen geradkettigen oder verzweigten, gesättigten oder ungesättigten Alkylrest mit 1 bis 8 Kohlenstoffatomen bedeuten, wobei die Alkylreste auch substituiert sein können durch Hydroxy, $C_1$—$C_4$-Alkoxy oder eine Aminogruppe der Formel

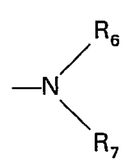

,

wobei $R_6$ und $R_7$ gleich oder verschieden sind und Wasserstoff oder oder einen geradkettigen oder verzweigten Alkylrest mit 1 bis 6 Kohlenstoffatomen, oder zusammen mit dem Stickstoffatom einen heterocyclischen Ring mit bis zu 7 Kohlenstoffatomen darstellen, wobei die Alkylreste $R_4$ und $R_5$ auch gemeinsam mit dem Stickstoffatom einen 5- bis 8-gliedrigen Ring bilden können, und der heterocyclische Ring an einem Kohlenstoffatom substituiert sein kann durch eine $C_1$—$C_6$-Alkyl-, $C_1$—$C_4$-Alkoxy-, Hydroxy-, Carboxy- oder $C_1$—$C_4$-Alkoxycarbonylgruppe und worin eines der Kohlenstoffatome durch ein Sauerstoff-, Schwefel- oder Stickstoffatom ersetzt sein kann, wobei das letzte substituiert sein kann durch Wasserstoff, die Thienyl-, Furyl-, Pyridyl- oder Formylgruppe, eine $C_3$—$C_8$ Alkenyloxycarbonyl- oder $C_3$—$C_8$-Alkinyloxycarbonylgruppe, eine gegebenenfalls durch Hydroxy- oder $C_1$—$C_4$-Alkoxygruppen substituierte $C_1$—$C_6$-Alkoxycarbonylgruppe, einen Phenylrest, der einfach oder mehrfach substituiert sein kann durch die $C_1$—$C_4$-Alkyl-, $C_1$—$C_4$-Alkoxy-, Methylendioxy-, Hydroxy-, Nitro- oder Aminogruppe oder Halogen, und wobei das Wasserstoffatom am Stickstoff weiterhin ersetzt sein kann durch den Rest —$COR_8$, worin $R_8$ einen Thienyl-, Furyl-, Pyridylrest oder einen gegebenenfalls wie oben angegebenen substituierter Phenylrest, oder eine $C_1$—$C_6$-Alkylgruppe bedeutet, die ihrerseits substituiert sein kann durch die Hydroxy-, $C_1$—$C_4$-Alkoxy-, $C_1$—$C_8$-Dialkylamino-, Äthylendioxy-, Trimethylendioxygruppe oder einen gegebenenfalls wie oben angegebenen substituierten Phenylrest. Weiterhin kann $R_3$ eine Carbamidgruppe der Formel

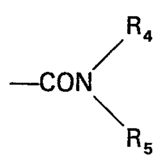

sein, worin $R_4$ und $R_5$ die obengenannte Bedeutung haben, sowie deren physiologisch verträglichen Salze.

2. Verfahren zur Herstellung von Verbindungen der allgemeinen Formel I, dadurch gekennzeichnet, daß man

a) Verbindungen der allgemeinen Formel II

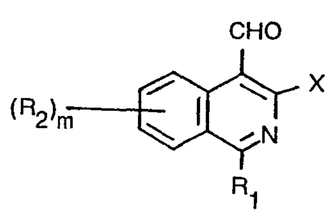

(II)

worin X, $R_1$, $R_2$ und m die zur allgemeinen Formel I genannte Bedeutung haben, zu Verbindungen der allgemeinen Formel I oxidiert, worin $R_3$ eine Carboxylgruppe ist. Diese können gegebenenfalls mit einem Amin

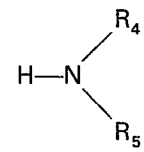 ,

worin $R_4$ und $R_5$ die zur allgemeinen Formel I genannte Bedeutung haben, zu Verbindungen der allgemeinen Formel I, in denen $R_3$ eine Carbamidgruppe der Formel

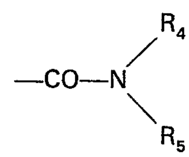

darstellt, umgesetzt werden,

b) Verbindungen der allgemeinen Formel III

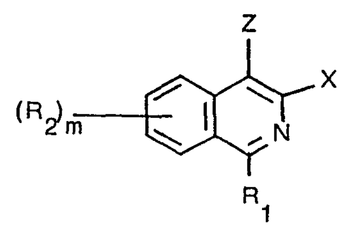

(III)

worin Z eine Formyl-, Nitril-, Carboxy-, Halogencarboxyl- oder eine Alkylcarboxylgruppe mit 1 bis 7 Kohlenstoffatomen bedeutet und X, $R_1$, $R_2$ und m die zur allgemeinen Formel I genannte Bedeutung haben, zu Verbindungen der allgemeinen Formel I reduziert, worin $R_3$ eine Hydroxymethylgruppe darstellt,

c) Verbindungen der allgemeinen Formel IV

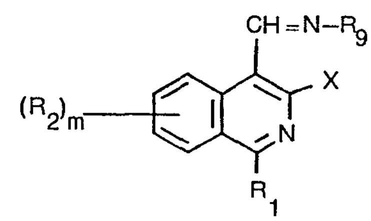

(IV)

worin X, $R_1$, $R_2$ und m die zur allgemeinen Formel I genannte Bedeutung haben, zu Verbindungen der allgemeinen Formel I reduziert, worin $R_3$ die Methylenaminogruppe der Formel —$CH_2$—NH—$R_9$ und $R_9$ der Rest

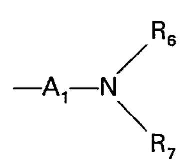

sein soll, wobei $A_1$ eine geradhaltige oder verzweigte $C_2$—$C_6$-Alkylengruppe darstellt, die durch eine Hydroxy- oder $C_1$—$C_4$-Alkoxygruppe substituiert sein kann, $R_6$ und $R_7$ die zur

allgemeinen Formel I genannte Bedeutung haben, darstellt,

d) Verbindungen der allgemeinen Formel V

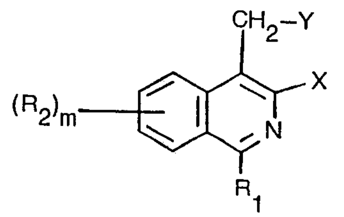

(V)

worin Y gleich Chlor, Brom oder Hydroxyl und X, $R_1$, $R_2$ und m die zur allgemeinen Formel I genannte Bedeutung haben, mit einem Amin der Formel

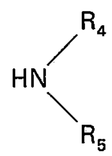

,

worin $R_4$ und $R_5$ die zur allgemeinen Formel I genannte Bedeutung haben, worin $R_3$ gleich

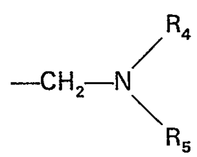

ist, umsetzt,

e) Verbindungen der allgemeinen Formel VI

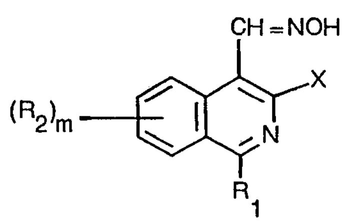

(VI)

worin X, $R_1$, $R_2$ und m die zur allgemeinen Formel I genannte Bedeutung haben, mit wasserabspaltenden Mitteln in Verbindungen der allgemeinen Formel I überführt, worin $R_3$ eine Nitrilgruppe darstellt,

f) Verbindungen der allgemeinen Formel VII

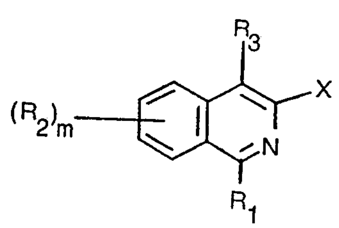

(VII)

worin X, $R_1$, $R_2$, $R_3$ und m die zur allgemeinen Formel I genannte Bedeutung haben mit der Maßgabe, daß in je einem oder beiden Resten $R_1$ und $R_3$ eine sekundäre Aminogruppe enthalten ist, mit einem Alkylierungsmittel der Formel $Y—R_{10}$, worin Y Jod, Chlor oder Brom, und $R_{10}$ einen geradkettigen oder verzweigten $C_1—C_6$-Alkylrest, der durch Hydroxy-$C_1—C_4$-Alkoxy, $C_1—C_4$-Dialkylamino, Äthylendioxy, Trimethylendioxy oder auch gegebenenfalls entsprechend substituiertes Phenyl substituiert sein kann, oder einen $C_3—C_8$-Alkenyl- oder $C_3—C_8$-Alkinylrest bedeutet, oder mit einem Chlorameisensäureester der Formel $Cl—CO_2—(C_1—C_4)$-alkyl, wobei die Alkylreste Hydroxy- oder $C_1—C_4$-Alkoxygruppen tragen oder mit einer Verbindung der Formel $Cl—COR_8$, worin $R_8$ die zur allgemeinen Formel I genannte Bedeutung hat, umsetzt,

g) Verbindungen der allgemeinen Formel I

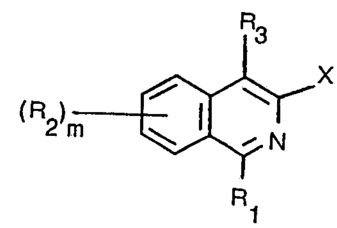

(I)

worin X, $R_1$, $R_2$, $R_3$ und m die zur allgemeinen Formel I genannte Bedeutung haben und sofern $R_1$ den Phenylring darstellt, diesen Phenylring nachträglich entsprechend substituiert.

3. Pharmazeutische Zubereitung, dadurch gekennzeichnet, daß sie eine Verbindung der allgemeinen Formel I enthält oder aus dieser besteht.

4. Verfahren zur Herstellung einer pharmazeutischen Zubereitung nach Anspruch 3, dadurch gekennzeichnet, daß man eine Verbindung der allgemeinen Formel I gegebenenfalls zusammen mit üblichen pharmazeutischen Trägern und/oder Stabilisatoren in eine für pharmazeutische Zwecke geeignete Darreichungsform bringt.

**Revendications**

1. Dérivés d'isoquinoléine de formule générale I

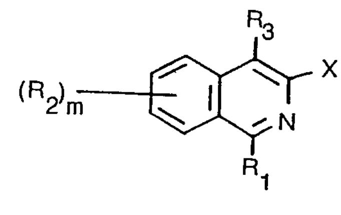

(I)

dans laquelle:

m est 1 ou 2,

X représente le brome ou le chlore,

$R_1$ représente un radical phényle qui éventuellement est mono- ou disubstitué avec un halogène, un groupe hydroxy, nitro, amino ou un groupe amino substitué par un ou deux radicaux hydrocarbones aliphatiques cycloaliphatiques ou aromatiques, et ayant de 2

à 18 atomes de carbone, l'atome d'azote pouvant être également inclus dans un noyau hétérocyclique, un groupe acylamino, alcoyle ou alcoxy ayant chacun de 1 à 6 atomes de carbone, un groupe benzyloxy ou trifluorométhyle, et représente ou représente un radical pyridyle ou thiényle,

$R_2$ représente l'hydrogène, un halogène, un groupe hydroxy, alcoyle ou alcoxy ayant de 1 à 6 atomes de carbone, un groupe nitro, amino, benzyloxy, méthylènedioxy, ou éthylènedioxy,

$R_3$ représente un groupe carboxy, cyano, hydroxyméthyle, alcoxyméthyle ayant de 1 à 6 atomes de carbone, un groupe aminométhyle de formule

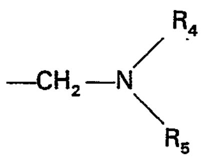 ,

où $R_4$ et $R_5$ sont identiques ou différents et représentent l'hydrogène ou un radical alcoyle saturé ou insaturé, à chaîne droite ou ramifiée ayant de 1 à 8 atomes de carbone, les radicaux alcoyle pouvant également être substitués par un groupe hydroxy, alcoxy en $C_1$—$C_4$ ou un groupe amino de formule

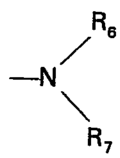 ,

où $R_6$ et $R_7$ sont identiques ou différents et représentent l'hydrogène ou un radical alcoyle à chaîne droite ou ramifiée ayant de 1 à 6 atomes de carbone, ou peuvent former ensemble avec l'atome d'azote un noyau hétérocyclique ayant jusqu'à 7 atomes de carbone, auquel cas les radicaux alcoyle $R_4$ et $R_5$ peuvent également former avec l'atome d'azote un noyau de 5 à 8 chaînons et le noyau hétérocyclique peut être substitué sur un atome de carbone par un groupe alcoyle en $C_1$—$C_6$, alcoxy en $C_1$—$C_4$, hydroxy, carboxy ou alcoxy($C_1$—$C_4$)-carbonyle et où un des atomes de carbone peut être remplacé par un atome d'oxygène, de soufre ou d'azote, auquel cas le dernier peut être substitué par l'hydrogène, le groupe thiényle, furyle, pyridyle ou formyle, un groupe alcényl($C_3$—$C_8$)-oxycarbonyle ou alcynyl($C_3$—$C_8$)-oxycarbonyle, un groupe alcoxy($C_1$—$C_6$)-carbonyle éventuellement substitué par des groupes hydroxy ou alcoxy en $C_1$—$C_4$, un radical phényle qui peut être une ou plusieurs fois substitué par un groupe alcoyle en $C_1$—$C_4$, alcoxy en $C_1$—$C_4$, méthylènedioxy, hydroxy, nitro ou amino, ou un halogène, et auquel cas l'atome d'hydrogène sur l'azote peut en outre être remplacé par le radical —$COR_8$ où $R_8$ représente un radical thiényle, furyle, pyridyle ou un radical phényle éventuellement substitué comme indiqué ci-dessus, ou un groupe alcoyle en $C_1$—$C_6$ qui de son côté peut être substitué par un groupe hydroxy, alcoxy en $C_1$—$C_4$, dialcoylamino en $C_1$—$C_8$, éthylènedioxy, triméthylènedioxy ou un radical phényle éventuellement substitué comme indiqué ci-dessus, $R_3$ peut en outre être un groupe carbamoyle de formule

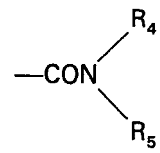 ,

où $R_4$ et $R_5$ ont les significations indiquées ci-dessus,

et leurs sels physiologiquement acceptables.

2. Procédé de préparation de composés de formule générale I, caractérisé en ce qu'il consiste

a) à oxyder des composés de formule générale II

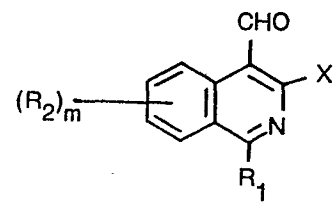 (II)

dans laquelle X, $R_1$, $R_2$ et m ont les significations indiquées pour la formule générale I, en des composés de formule générale I où $R_3$ est un groupe carboxy, et éventuellement à faire réagir les composés obtenus avec une amine

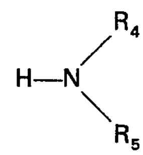

où $R_4$ et $R_5$ ont les significations indiquées pour la formule I, pour obtenir des composés de formule générale I dans lesquels $R_3$ représente un groupe carbamoyle de formule

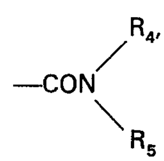 ,

b) à reduire des composés de formule générale III

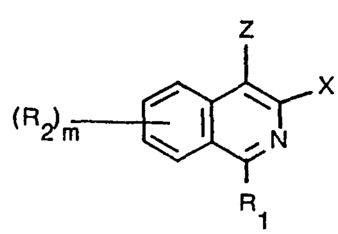 (III)

dans laquelle Z représente un groupe formyle, nitrile, carboxy, halocarboxy, ou alcoylcarboxy, avec de 1 à 7 atomes de carbone, et X, $R_1$, $R_2$ et m ont les significations indiquées pour la formule générale I, en des composés de formule générale I où $R_3$ représente un groupe hydroxyméthyle,

c) à réduire des composés de formule générale IV

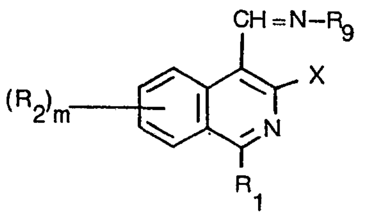

(IV)

dans laquelle X, $R_1$, $R_2$ et m ont les significations indiquées pour la formule générale I, en des composé de formule générale I où $R_3$ représente le groupe méthylèneamino de formule $-CH_2-NH-R_9$ ou $R_9$ est un radical

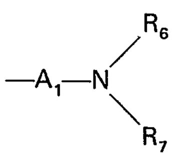

$A_1$ représentant un groupe alkylène en $C_2-C_4$ à chaîne droite ou ramifiée qui peut être substitué par un groupe hydroxy ou alcoxy en $C_1-C_4$, et $R_6$ et $R_7$ ont les significations indiquées pour la formule générale I,

d) à faire réagir des composés de formule générale V

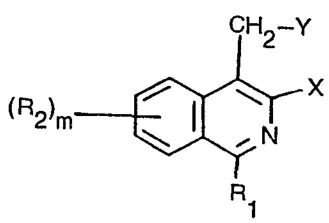

(V)

dans laquelle Y est le chlore, le brome ou un groupe hydroxyle et X, $R_1$, $R_2$ et m ont les significations indiquées pour la formule générale I, avec une amine de formule

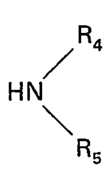

où $R_1$ et $R_2$ ont les significations indiquées pour la formule générale I, pour obtenir des composés de formule générale I ou $R_3$ est le groupe

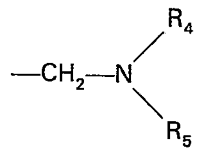

e) à convertir des composés de formule générale VI

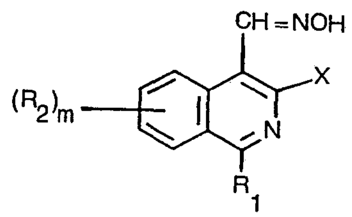

(VI)

dans laquelle X, $R_1$, $R_2$ et m ont les significations indiquées pour la formule générale I, avec des agents déshydratants, en des composés de formule générale I où $R_3$ représente un groupe nitrile,

f) à faire réagir des composés de formule générale VII

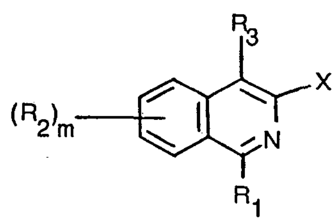

(VII)

dans laquelle X, $R_1$, $R_2$, $R_3$ et m ont les significations indiquées pour la formule générale I, à la condition que l'un ou les deux groupes $R_1$ et $R_3$ contienne un groupe amino secondaire, avec un agent d'alkylation de formule $Y-R_{10}$, où Y représente l'iode, le chlore ou le brome et $R_{10}$ représente un radical alcoyle en $C_1-C_6$, à chaîne droite ou ramifiée, qui peut être substitué par un groupe hydroxy, alcoxy en $C_1-C_4$, dialcoylamino en $C_1-C_4$, éthylènedioxy, triméthylènedioxy ou également phényle éventuellement substitué en conséquence, ou un radical alcényle en $C_3-C_8$ ou alcynyle en $C_3-C_8$, ou avec un ester de l'acide chloroformique de formule $Cl-CO_2$-alcoyle en $C_1-C_4$, auquel cas le radical alcoyle peut porter des groupes hydroxy ou alcoxy en $C_1-C_4$, ou avec un composé de formule $ClCOR_8$ où $R_8$ a la signification indiquée pour la formule générale I, et

g) dans la mesure où $R_1$ représente le noyau phényle dans des composés de formule générale I

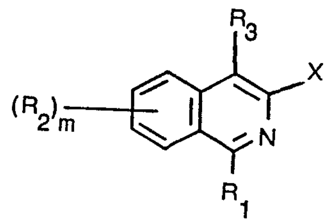

(I)

dans laquelle X, $R_1$, $R_2$, $R_3$ et m ont les significations indiquées pour la formule générale I, à substituer ultérieurement ce noyau phényle en conséquence.

3. Composition pharmaceutique caractérisée en ce qu'elle contient un composé de formule générale I ou consiste en celui-ci.

4. Procédé de préparation d'une composition pharmaceutique selon la revendication 3, caractérisé en ce qu'on met un composé de formule générale I éventuellement associé à des véhicules et/ou des stabilisants pharmaceutiques usuels, sous une forme de présentation appropriée pour un usage pharmaceutique.

## Claims

1. Isoquinoline derivatives of the general formula I

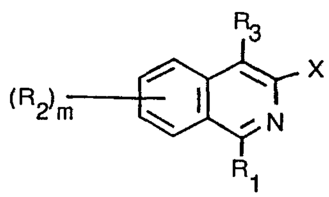

(I)

wherein
m is 1 or 2,
X is a bromine or chlorine,
$R_1$ is phenyl optionally mono- or disubstituted by halogen; hydroxy; nitro; unsubstituted amino or amino substituted by one or two aliphatic, cycloaliphatic or aromatic hydrocarbon radicals and having two to eighteen carbon atoms, the nitrogen atom of said amino being optionally included in a heterocyclic ring; acylamino; alkyl or alkoxy each having one to six carbon atoms; benzyloxy or trifluoromethyl; or $R_1$ is pyridyl or thienyl;
$R_2$ is hydrogen; halogen; hydroxy; alkyl or alkoxy having one to six carbon atoms; nitro; amino; benzyloxy; methylenedioxy or ethylenedioxy;
$R_3$ is carboxyl; cyano; hydroxymethyl; alkoxymethyl having from 1 to six carbon atoms; aminomethyl of the formula

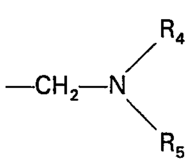

,

wherein $R_4$ and $R_5$ are identical or different and denote hydrogen or straight chain or branched, saturated or unsaturated alkyl having from 1 to 8 carbon atoms and being optionally substituted by hydroxy, $C_{1-4}$ alkoxy or amino of the formula

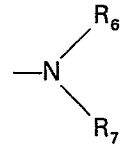

,

wherein $R_6$ and $R_7$ are identical or different and denote straight chain or branched alkyl having from 1 to 6 carbon atoms or, when taken together, a heterocyclic ring having up to 7 carbon atoms.

The alkyl radicals $R_4$ and $R_5$ may alternatively form a five- to eight-membered ring, when taken together with the nitrogen atom, the heterocyclic ring being optionally substituted at one carbon atom by $C_1$—$C_6$ alkyl, $C_1$—$C_4$ alkoxy, hydroxy, carboxy or $C_1$—$C_4$ alkoxycarbonyl and one of the carbon atoms of the heterocyclic ring being optionally replaced by an oxygen, sulfur or nitrogen atom, the latter being optionally substituted by hydrogen, thienyl, furyl, pyridyl or formyl, $C_3$—$C_8$ alkenyloxycarbonyl or $C_3$—$C_8$ alkinyloxycarbonyl, $C_1$—$C_6$ alkoxycarbonyl optionally substituted by hydroxy or $C_1$—$C_4$ alkoxy, phenyl optionally substituted once or several times by $C_1$—$C_4$ alkyl, $C_1$—$C_4$ alkoxy, methylenedioxy, hydroxy, nitro, amino or halogen and the hydrogen atom at the nitrogen atom being further substituted optionally by —$COR_8$ wherein $R_8$ denotes thienyl, furyl, pyridyl or phenyl, optionally substituted as specified above, $C_1$—$C_6$ alkyl, the latter being optionally substituted by hydroxy, $C_1$—$C_4$ alkoxy, $C_1$—$C_6$ dialkylamino, ethylene dioxy, trimethylenedioxy or phenyl optionally substituted as specified above. $R_3$ may further be a carboxylic amide group of the formula

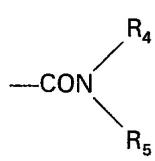

,

wherein $R_4$ and $R_5$ are defined as above, and physiologically acceptable salts thereof.

2. Process for the preparation of compounds of the general formula I characterized by

a) oxidizing compounds of the general formula II

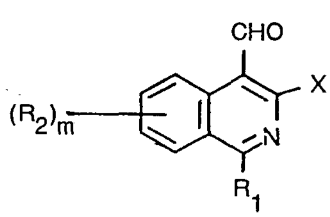

(II)

wherein X, $R_1$, $R_2$ and m are defined as in general formula I, to give compounds of the general formula I wherein $R_3$ is carboxyl. The latter compounds may be reacted with an amine of the formula

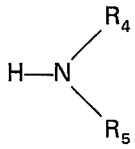

wherein $R_4$ and $R_5$ are defined as in general formula I, to give compounds of the formula I wherein $R_3$ is carboxylic amide of the formula

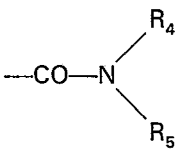 ,

b) reducing compounds of the general formula III

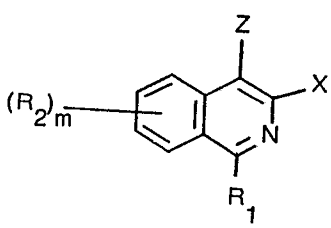

(III)

wherein Z is formyl, nitril, carboxy, halogencarboxyl or alkylcarboxyl having from 1 to 7 carbon atoms and X, $R_1$, $R_2$ and m are defined as in general formula I, to give compounds of the general formula I wherein $R_3$ is hydroxymethyl,

c) reducing compounds of the general formula IV

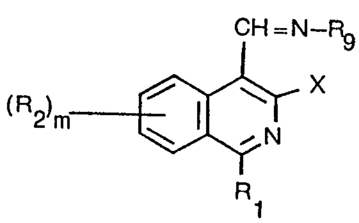

(IV)

wherein X, $R_1$, $R_2$ and m are defined as in general formula I, to give compounds of the general formula I wherein $R_3$ is methylenamino of the formula $-CH_2-NH-R_9$ with $R_9$ denoting the radical

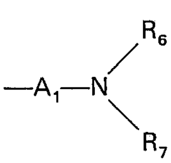

wherein $A_1$ denotes straight chain or branched $C_2-C_6$ alkylene, optionally substituted by hydroxy or $C_1-C_4$ alkoxy, and $R_6$ and $R_7$ are defined as in general formula I,

d) reacting compounds of the general formula V

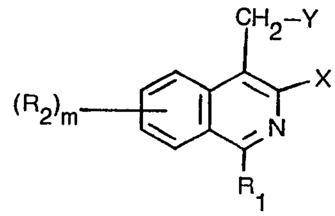

(V)

wherein Y is chlorine, bromine or hydroxy and X, $R_1$, $R_2$ and m are defined as in general formula I, with an amine of the formula

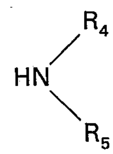

with $R_4$ and $R_5$ being defined as in general formula I, $R_5$ being the group

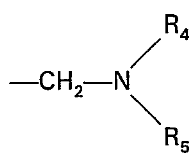 ,

e) converting compounds of the general formula VI

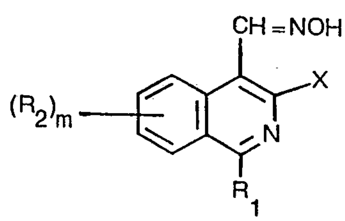

(VI)

wherein X, $R_1$, $R_2$ and m are defined as in general formula I, with agents splitting off water into compounds of the general formula I wherein $R_3$ is nitril,

f) reacting compounds of the general formula VII

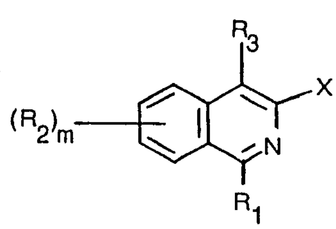

(VII)

wherein X, $R_1$, $R_2$, $R_3$ and m are defined as in general formula I, provided that in one of the radicals or in both of the radicals $R_1$ and $R_3$ a secondary amino group is included, with an alkylation agent of the formula Y—$R_{10}$, wherein Y is iodine, chlorine or bromine, and $R_{10}$ is straight chain or branched $C_1$—$C_6$ alkyl, optionally substituted by hydroxy, $C_1$—$C_4$ alkoxy, $C_1$—$C_4$ dialkylamino, ethylenedioxy, trimethylenedioxy or optionally correspondingly substituted phenyl, or $R_{10}$ is $C_3$—$C_8$ alkenyl or $C_3$—$C_8$ alkinyl, or reacting compounds of the formula VII with a chloroformate of the formula Cl—$CO_2$($C_1$—$C_4$)alkyl, the alkyl radicals being substituted by hydroxy or $C_1$—$C_4$ alkoxy, or reacting compounds of the formula VII with a compound of the formula Cl—$COR_8$ with $R_8$ being defined as in general formula I,

g) compounds of the general formula I

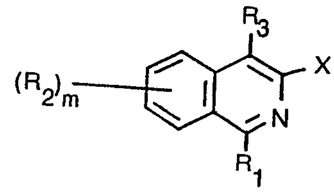

(I)

wherein X, $R_1$, $R_2$, $R_3$ and m are defined as in general formula I, subsequently correspondingly substituting phenyl, if $R_3$ denotes phenyl.

3. Pharmaceutical preparations, containing a compound of the general formula I or consisting thereof.

4. Process for the manufacture of a pharmaceutical preparation as claimed in claim 3, which comprises bringing a compound of the formula I, optionally in conjunction with usual pharmaceutical carriers and/or stabilizers, into a form application suitable for pharmaceutical.